(19) Europäisches Patentamt
European Patent Office
Office européen des brevets

(11) EP 1 862 147 A1

(12) **EUROPEAN PATENT APPLICATION**

(43) Date of publication:
05.12.2007 Bulletin 2007/49

(51) Int Cl.:
*A61F 2/16* (2006.01)

(21) Application number: 07108530.2

(22) Date of filing: 21.05.2007

(84) Designated Contracting States:
AT BE BG CH CY CZ DE DK EE ES FI FR GB GR HU IE IS IT LI LT LU LV MC MT NL PL PT RO SE SI SK TR
Designated Extension States:
AL BA HR MK YU

(30) Priority: 31.05.2006 US 444113

(71) Applicant: Alcon, Inc.
6331 Hünenberg (CH)

(72) Inventors:
• Hong, Xin
Arlington, TX 76017 (US)
• Karakelle, Mutlu
Fort Worth, TX 76132 (US)

• Zhang, Xiaoxiao
Fort Worth, TX 76132 (US)
• Weinschenk, Joseph I.
Fort Worth, TX 76132 (US)
• Carson, Daniel R.
Fort Worth, TX 76133 (US)

(74) Representative: Hanna, Peter William Derek et al
Hanna, Moore & Curley
13 Lower Lad Lane
Dublin 2 (IE)

Remarks:
A request for correction of the description has been filed pursuant to Rule 88 EPC. A decision on the request will be taken during the proceedings before the Examining Division (Guidelines for Examination in the EPO, A-V, 3.).

(54) **Correction of chromatic abberations in intraocular lenses**

(57)  In one aspect, the present invention provides an intraocular lens (10) that includes a posterior optic (12) and anterior optic (14). The optics have different chromatic dispersions adapted to cooperatively provide compensation for natural chromatic aberrations of the eye over a wavelength range of interest, e.g., over a wavelength range of 400 nm to 700 nm.

## Fig. 1

EP 1 862 147 A1

**Description**

**FIELD OF THE INVENTION**

The present invention relates generally to ophthalmic lenses, and more particularly, to ophthalmic lenses that provide compensation for the chromatic aberrations of the eye.

**BACKGROUND**

The refractive power of the human eye varies as a function of the wavelength of incident radiation such that the eye is more myopic for blue light and more hyperopic for red light. For example, the optical power of the eye can vary by about 2 Diopters (D) over a wavelength range of 400 nm to 700 nm. This change of optical power as a function of wavelength, which is commonly known as chromatic aberration or chromatic defocus, can degrade the image contrast. Such chromatic aberrations can adversely affect the optical performance of ocular ophthalmic lenses and implants, which are utilized by a growing segment of the population. In addition, ocular ophthalmic lenses and implants generally exhibit chromatic aberrations of their own, which can further degrade their optical performance.

Accordingly, there is a need for enhanced ophthalmic lenses, such as intraocular lenses, that provide better optical performance for polychromatic incident light.

**SUMMARY**

The present invention provides an intraocular lens in accordance with claims which follow, and is generally directed to multi-element intraocular lenses (IOLs) that can compensate for natural chromatic aberrations of the eye, particularly the longitudinal chromatic aberration. More specifically, various parameters of the lens elements, such as their chromatic dispersions (variations of index of refraction as a function of wavelength) as well as surface curvatures, are adapted so that those elements collectively provide a desired degree of chromatic aberration correction.

In one aspect, the invention provides an intraocular lens that includes a posterior optic and an anterior optic. The optics have different chromatic dispersions adapted to cooperatively provide compensation for natural chromatic aberrations of the eye over a wavelength range of interest. In other words, the optics are adapted to at least partially correct the effects of the chromatic aberrations exhibited by the eye (variations of focal lengths for different wavelength components of incident light).

In a related aspect, the wavelength range over which the compensation of the chromatic aberration is achieved can be centered about 570 nm, and extend from about 400 nm to about 700 nm. By way of example, the optics can be adapted to collectively provide a chromatic aberration correction in a range of about 0.5 to about 3.5 Diopters over a wavelength range of about 400 nm to about 700 nm.

In another aspect, one of the optics provides a positive optical power and the other provides a negative optical power. In many cases, the optics jointly provide a total optical power in a range of about 6 Diopters to about 34 Diopters.

The optics can be axially separated, or can be in contact via two of their surfaces. More generally, the distance between the optics (e.g., separation between centers of the optics) can be in a range of about 0 to about 5 millimeters (e.g., in a range of about 0.1 to about 5 mm). Further, an optical axis of one optic can be preferably substantially coincident with an optical axis of the other optic.

In a related aspect, both optics are formed of a biocompatible material. Some examples of such materials include, without limitation, soft acrylic polymers with sub-ambient glass transition temperatures, hydrogel, polymethylmethacrylate, polysulfone, polystyrene, cellulose acetate butyrate or other biocompatible polymeric materials having a requisite index of refraction for a particular application. By way of example, in some cases, one optic is formed of polymethylmethacrylate (PMMA) and the other is formed of polysulfone. In another example, one optic is formed of a soft acrylic material (a cross-linked copolymer of 2-phenylethyl acrylate and 2-phenylethyl methacrylate) known as Acrysof and the other optic is formed of a cross-linked terpolymer of ethyl acrylate, ethylmethacrylate and 2,2,2 - trifluoroethyl methacrylate commonly known as Sensar.

In another aspect, an intraocular lens is disclosed that includes a posterior optic exhibiting a chromatic dispersion over a wavelength range, and an anterior optic exhibiting a different chromatic dispersion over that wavelength range. The optics include a plurality of curved surfaces having curvatures that are adapted to generate, together with the difference in the chromatic dispersions of the optics, chromatic focal shifts for compensating chromatic aberrations of the eye over that wavelength range.

In a related aspect, the chromatic focal shifts provide a correction in a range of about 0.5 Diopters to about 3.5 Diopters over the wavelength range of interest, which can extend, e.g., from about 400 nm to about 700 nm.

In another aspect, an ophthalmic lens system is disclosed that includes a posterior lens and an anterior lens. A diffractive pattern is disposed on a surface of one of those lenses such that the lenses cooperatively provide a near focus and a far focus. The anterior and posterior lenses exhibit different chromatic dispersions adapted to compensate for chromatic aberration of the eye at the far focus over a wavelength range, e.g., over a wavelength range of about 400 nm to about 700 nm.

In a related aspect, the optical power associated with the far focus is in a range of about 6 D and 34 D, and the diffractive pattern provides an add power in a range of about 1 to about 6 D.

In another aspect, the invention provides an intraocular lens system that includes a posterior optic and an anterior optic. The optics are movably coupled to one another so as to allow movement of at least one optic, in response to application of a compressive force thereto, relative to the other. Further, the optics have different chromatic dispersions adapted to cooperatively provide compensation for natural chromatic aberrations of the eye over a wavelength range.

In a related aspect, the above lens system provides an accommodation (pseudo-accommodation) in a range of about 1 to about 6 Diopters when implanted in a patient's eye.

In another aspect, in the above pseudo-accommodative lens system, the optics are adapted to collectively provide a chromatic aberration correction in a range of about 0.5 to about 3.5 Diopters over a wavelength range extending from about 400 nm to about 700 nm.

In another aspect, in the above pseudo-accommodative lens system, one optic provides a positive optical power (e.g., in a range of about 20 D to about 80 D) while the other optic provides a negative optical power (e.g., in a range of about -5 D to about -60 D). Further, the optics can be formed of two different materials, preferably biocompatible, whose Abbe numbers differ by at least about 10. For example, one optic can be formed of PMMA and the other of a soft acrylic material. In another example, one of the optics can be formed of a cross-linked copolymer of 2-phenylethyl acrylate and 2-phenylethyl methacrylate and the other optic can be formed of a cross-linked terpolymer of ethyl acrylate, ethylmethacrylate, and 2,2,2-trifluoroethyl methacrylate.

Further understanding of the invention can be obtained by reference to the following detailed description in conjunction with the associated drawings, described briefly below.

BRIEF DESCRIPTION OF THE DRAWINGS

FIGURE 1 is a schematic side view of an IOL according to one embodiment of the invention,

FIGURE 2 is a schematic top plan view of the IOL of FIGURE 1,

FIGURE 3 is a schematic side view of an IOL according to another embodiment of the invention,

FIGURE 4 depicts a polychromatic modulation transfer function (MTF) for a pseudophakic model eye having an aspheric monofocal implant,

FIGURE 5 depicts a polychromatic modulation transfer function (MTF) for a pseudophakic model eye having a spherical monofocal implant,

FIGURE 6 schematically depicts a theoretical model eye in which a doublet lens according to one embodiment of the invention is incorporated to provide full correction of the chromatic aberration,

FIGURE 7 depicts a polychromatic MTF calculated for the model eye of FIGURE 6,

FIGURE 8 depicts a polychromatic MTF calculated for a model eye in which a doublet lens according to one embodiment of the invention is incorporated to provide partial correction of the chromatic aberration,

FIGURE 9 depicts a polychromatic MTF calculated for an average model eye in which a doublet lens according to another embodiment of the invention is incorporated,

FIGURE 10 is a schematic side view of an ophthalmic lens according to another embodiment of the invention that includes a diffractive pattern disposed on a surface thereof,

FIGURE 11A is a schematic cross-sectional view of an IOL according to another embodiment of the invention,

FIGURE 11B is a schematic top view of the anterior optic of the IOL of FIGURE 11A, and

FIGURE 11C is a schematic cross-sectional view of the anterior optic of the IOL of FIGURE 11A.

**DETAILED DESCRIPTION**

The present invention generally provides ophthalmic lenses that are capable of compensating, either fully or partially, for the natural chromatic aberrations of the eye. Although the following embodiments are primarily directed to intraocular lenses, the teachings of the invention can be applied to a variety of lenses and ocular implants, such as, contact lenses. Further, the terms "intraocular lens" and its abbreviation "IOL" are used herein interchangeably to describe lenses that are implanted into the interior of an eye to either replace the eye's natural lens or to otherwise augment vision regardless of whether or not the natural lens is removed.

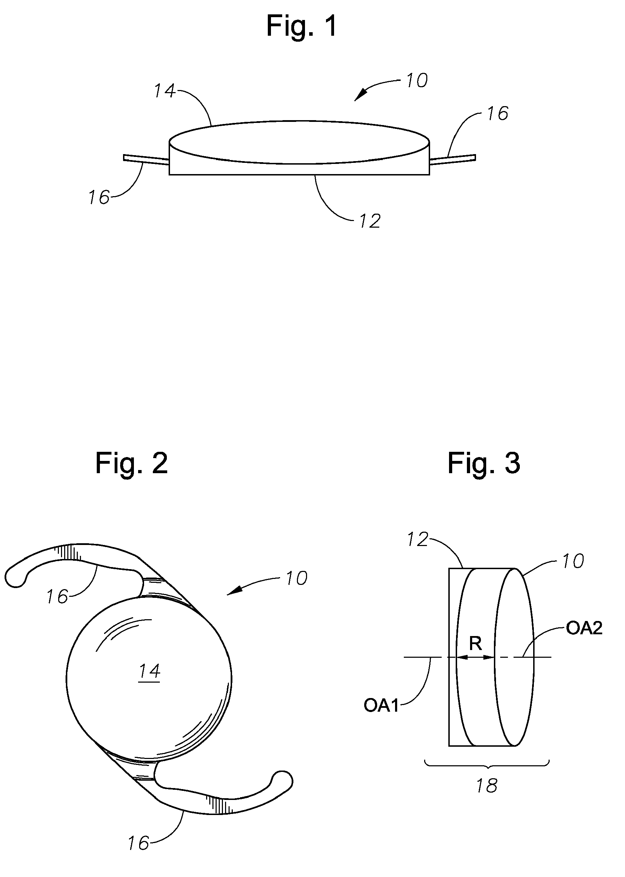

With reference to FIGURES 1 and 2, an intraocular lens (IOL) 10 according to an exemplary embodiment of the invention includes a posterior lens 12, an anterior lens 14 and a plurality of fixation members or haptics 16 that facilitate placing the IOL in a patient's eye. In this embodiment, the posterior lens 12 is a plano-concave lens while the anterior lens 14 is a biconvex lens. In other embodiments, alternative lens configurations (e.g., plano-convex) can be employed for either of the posterior and/or anterior lens. Further, lens 12 has preferably a negative power (e.g., a power in a range of about -5 D to about -60 D) and lens 14 has preferably a positive power (e.g., a power in a range of about 20 D to about 80 D), although the differences in power can also be reversed. The optical power of the combined lenses, that is the optical power of the IOL 10, can be in a range of about -15 to about 50 D, and preferably in a range of about 6 D to about 34 D.

Although in this embodiment the lenses 12 and 14 are in contact with one another, in other embodiments they can be separated along an axial direction by a distance, e.g., less than about 5 mm. In many embodiments in which the lenses are axially separated, they are preferably positioned relative to one another such that their optical axes are aligned. By way of example, FIGURE 3 schematically depicts an ophthalmic lens 18 formed of lenses 12 and 14, which are axially separated from one another by a distance R in a range of about 0.1 to about 5 mm. An optical axis OA1 of the lens 12 is substantially aligned with an optical axis OA2 of the lens 14.

Referring again to FIGURES 1 and 2, lenses 12 and 14 are made of different materials that exhibit a difference in their chromatic dispersions, which is adapted to ensure that the chromatic aberration of the eye implanted with the lens 10 is minimized, or eliminated. As is known in the art, a variation of the refractive index of a material as a function of radiation wavelength is referred to as the dispersion of that material. One commonly employed measure of a material's dispersion (variation of refractive index with wavelength) is known as Abbe number (also known as V-number or constringence of a material), and is defined as follows:

$$V = \frac{n_D - 1}{n_F - n_C} \qquad \text{Eq. (1)}$$

where $n_D$, $n_F$ and $n_C$ represent the refractive indices of the material at wavelengths of 589.2 nm, 486.1 nm and 656.3 nm, respectively, that correspond to Fraunhofer D-, F-, and C-spectral lines. In general, materials having high values of V exhibit low dispersions. In many embodiments, the materials forming the lenses 12 and 14 have sufficiently different V numbers so as to minimize, and in some cases eliminate, the chromatic aberration of the IOL 10 and/or cause the net power of the IOL 10 to vary as function of incident light wavelength in a manner that would compensate for (counter) chromatic dependent refractive error of the eye.

By way of example, the chromatic aberration exhibited by the IOL can be in a range of about 0.5 D to about 3.5 D. Further, in some embodiments, the materials forming the lenses 12 and 14, and the relative power of the two lenses, are selected so as to compensate for natural chromatic aberrations of the eye over a wavelength range, e.g., a wavelengths range of about 400 nm to about 700 nm. The phrase "to compensate for natural chromatic aberrations of the eye," as used herein is intended to encompass not only those cases in which the chromatic aberrations are fully counteracted but also cases in which some residual chromatic aberrations remain, e.g., a residual longitudinal aberration less than about 50%. For example, the IOL 10 can provide a chromatic aberration correction in a range of about 1 to about 2 Diopters over a wavelength range of about 400 nm to about 700 nm.

The lenses 12 and 14 can be formed from a variety of materials, which are preferably biocompatible. By way of example, one lens (e.g., lens 14) can be made from polymethylmethacrylate (PMMA) (Abbe number V = 55) and the other lens (e.g., lens 12) can be made from polysulfone (Abbe number V = 30.87). Other suitable materials include, without limitation, soft acrylics, such as Acrysof (cross-linked copolymer of 2-phenylethyl acrylate and 2-phenylethyl methacrylate, (Abbe number V of about 37), polystyrene (Abbe number V = 30.87), polycarbonate (Abbe number V of 29.9), or cellulose acetate butyrate (Abbe number V in a range of about 80 to 84), and the materials disclosed in U.S. Patent No. 4,834,750, so long as the differences between the Abbe numbers of the materials forming the two lenses are sufficiently large (e.g., greater than about 10) to provide a desired chromatic compensation. For example, in one embodiment, lenses 12 and 14 can be made, respectively, from PMMA and Acrysof with the lens 12 having an optical power of about - 43.17 Diopters and the lens 14 having an optical power of about +64.17 Diopters.

In some embodiments, the lens 12 having a positive optical power can be formed of a material with low dispersion (high V number) and the lens 14 having a negative optical power can be formed of a material with higher dispersion (lower V number) such that the combined optical power of the two lenses is positive.

An achromatizing IOL of the invention can be employed for capsule implantation in an aphakic eye or for anterior or posterior implantation in a phakic eye.

By way of illustration, a prototype achromatizing doublet lens, such as the above lens 10, was theoretically designed by utilizing a model eye with an aspheric cornea characterized by a conic constant (cc) of -0.183 and OSLO™ lens design software, marketed by Lambda Research Corporation of Littleton, Mass., U.S.A. An aperture of about 4.5 mm at the pupil plane of the model eye was employed and the wavelengths of 550, 488 and 633 nm were weighted to approximate photopic response of the eye.

The following relation among the V numbers of the two lenses and their respective optical powers were utilized in these design examples:

$$\phi_2 = \frac{\phi_1 * V_2}{V_1} \quad \text{Eq. (2)}.$$

where $\phi_1$ and $\phi_2$ represent the optical powers of the two lenses, and $V_1$ and $V_2$ are the Abbe numbers of the materials from which the two lenses are formed.

In the illustrative design, the material of one lens was selected to have a low refractive index and a high Abbe number while the material of the other lens was selected to have a high refractive index and a low Abbe number. The lens formed of the material having a lower refractive index was chosen to have a positive optical power while the lens formed of the material having a higher refractive index was selected to have a negative optical power.

As a comparative reference for evaluating the achromatizing design, the chromatic properties of a pseudophakic model eye having an aspheric monofocal implant formed of soft acrylic material (a material used in an implant marketed as Acrysof™) were calculated. More specifically, the following model eye was set up in OSLO design software for these calculations:

**Table 1**

| Surface | Radius (mm) | Conic constant | Thickness (mm) | Material |
|---|---|---|---|---|
| 1 | 7.72 | -0.183 | 0.55 | Cornea |
| 2 | 6.5 | 0 | 3.05 | Aqueous |
| 3 | Infinity | 0 | 1.00 | Aqueous |
| 4 | 20.3 | -27.7 | 0.614 | AcrySof |
| 5 | -20.738 | 0 | 15.386 | Vitreous |
| 6 | Infinity | 0 | 2.94133 | Vitreous |
| 7 | Infinity | 0 | -0.02684 | Air |

As shown in the Table 2 below, the above pseudophakic eye exhibits a power change of 3.16 D over a wavelength range of 400 to 700 nm and a power change of 1.41 D over a wavelength range of 488 to 656 nm.

**Table 2**

| $\lambda$ (nm) | EFL | Optical Power (D) | Optical Power Difference from 550 $\lambda$ |
|---|---|---|---|
| 400 | 15.913 | 62.84 | -2.22 |
| 486 | 16.313 | 61.30 | -0.68 |
| 550 | 16.496 | 60.62 | 0 |
| 656 | 16.696 | 59.89 | 0.73 |
| 700 | 16.755 | 59.68 | 0.94 |

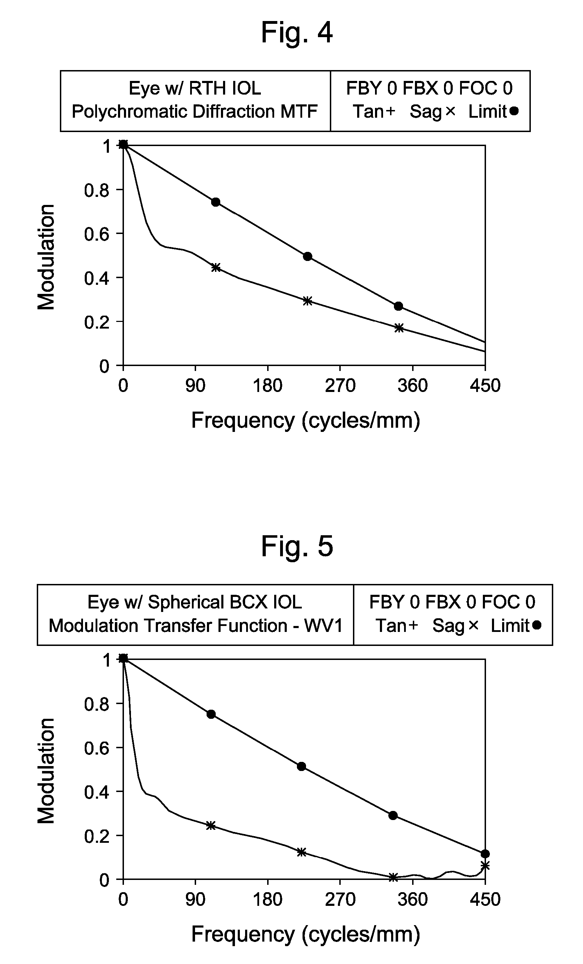

FIGURE 4 shows a polychromatic modulation transfer function (MTF) calculated for the above eye model for polychromatic incident radiation having wavelengths of 550, 488 and 656 nm (the upper curve is a diffraction limited MTF reference curve). As known in the art, the MTF associated with an optical system can be defined as a ratio of a contrast associated with an image of an object formed by the optical system relative to a contrast associated with the object. FIGURE 5 shows a similarly calculated MTF for a model eye having the same parameters as the above pseudopakhic model eye but without the asphericity associated with the anterior surface of the implant (again, the upper curve is a diffraction limited MTF reference curve). The MTF value at 100 lp/mm (line pairs per millimeter) is 0.252 for the eye model having the spherical implant and 0.438 for the eye model having the aspherical implant. The remaining reduction in MTF in the eye model having the aspherical implant is substantially due to chromatic aberrations.

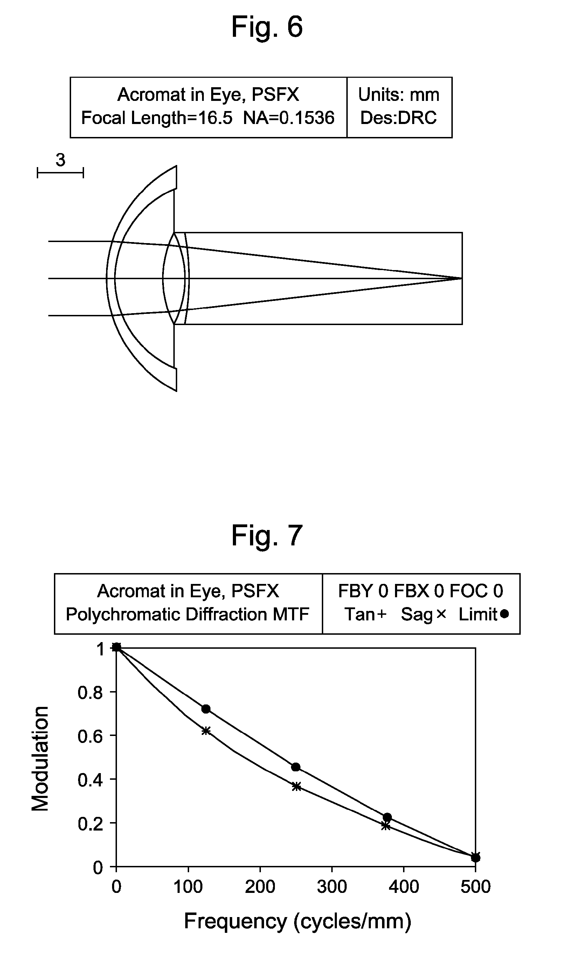

In the lens design example, the properties of an achromatic doublet lens having an equiconvex PMMA positive lens (with an optical power of 39 D) and a meniscus polysulfone (V = 22.5) lens (with an optical power of -19 D) in contact with a posterior surface of the PMMA lens were evaluated. This doublet lens was substituted for the monofocal IOL in the above eye model, as shown schematically in FIGURE 6, and its parameters were optimized (e.g., by adjusting the radii of curvature of one or more surfaces) to provide full chromatic correction. The thickness of the doublet was calculated to be about 1.7 mm.

FIGURE 7 shows a polychromatic MTF (wavelengths of 550 nm, 488, and 656 nm) calculated for an eye model in which the above doublet lens was incorporated at a pupil size of about 4.5 mm (the upper curve is a diffraction limited reference MTF). The MTF shows a value of about 0.69 at 100 lp/mm, thus illustrating a significant improvement in contrast relative to the reference eye model.

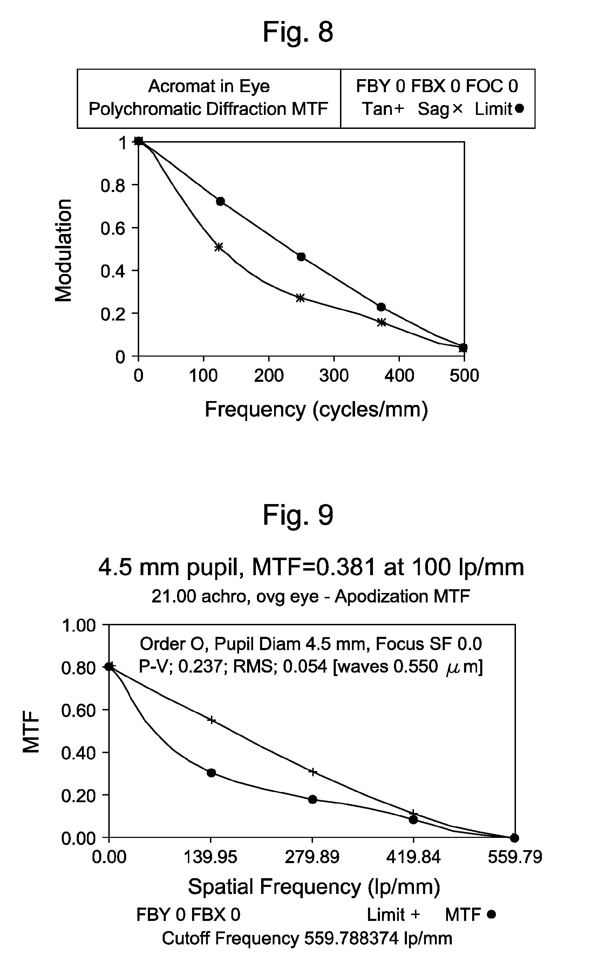

In another case, the PMMA/polysulfone lens was incorporated in the eye model, but the lens parameters were optimized to correct half of the chromatic aberration associated with the reference eye model. A polychromatic MTF associated with this case, shown in FIGURE 8, exhibits a value of about 0.59 at 100 lp/mm, which is less than the corresponding value obtained for the fully-corrected case, but still significantly greater than the respective value of the MTF for the reference eye model (the upper curve in the figure is a diffraction limited reference MTF).

In some embodiments, an achromatizing doublet includes a positive lens formed of a material utilized in commercially available lenses sold under trademark Acrysof (cross-linked copolymer of 2-phenylethyl acrylate and 2-phenylethyl methacrylate), and a negative lens formed of a material commonly known as Sensar (cross-linked terpolymer of ethyl acrylate, ethylmethacrylate, and 2,2,2-trifluoroethyl methacrylate). By way of illustration, the optical property of such an achromatizing multifocal doublet was simulated by computing the polychromatic MTF exhibited by an average eye model in which the doublet was incorporated. More specifically, a doublet lens having a negative Acrysof multifocal lens having a distance power of about -15 D and a positive Sensar lens having a power of about 35 D was incorporated in an average model eye, and a polychromatic MTF (wavelengths of 488 nm, 550 nm and 633 nm) was calculated at the focal plane of the model. The two lenses were configured to provide 1.26 D of chromatic aberration correction. FIGURE 9 shows the calculated polychromatic (wavelengths of 550, 488 and 633 nm) MTF for such a model eye incorporating the achromatic doublet (a pupil size of 4.5 mm was employed). The MTF at 100 lp/mm is about 0.381, which is much enhanced relative to an MTF of about 0.276 calculated for the model eye with a singlet lens formed of Acrysof lens material. The upper curve in the figure is a diffraction limited reference MTF.

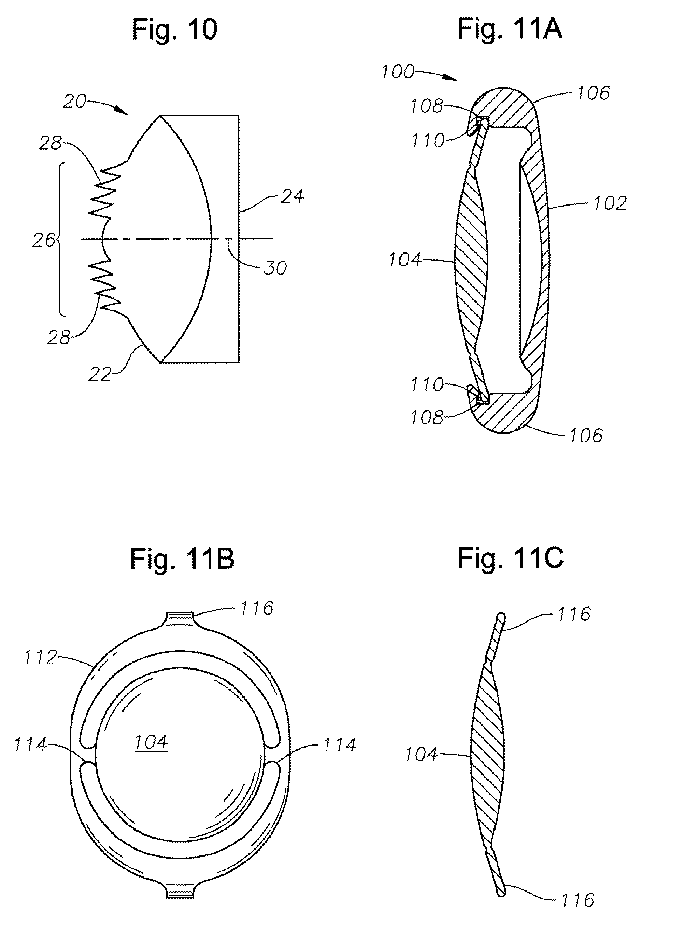

The teachings of the invention can also be applied to multi-focal ophthalmic lenses, such as, intraocular lenses that can provide both near and far vision. By way of example, FIGURE 10 schematically illustrates an ophthalmic lens (e.g., IOL) 20 in accordance with another embodiment of the invention that includes an anterior lens 22 and an posterior lens 24. In this embodiment, a surface of the anterior lens includes a diffraction pattern 26, which comprises a plurality of diffractive zones 28, for generating an add power (near focus). More particularly, the IOL 20 provides a far focus with an optical power, e.g., in a range of about 6 D to about 34 D, and a near focus with an optical add power, e.g., in a range of about 1 D to about 6 D. Each diffractive zone 28 is separated from an adjacent one by a step height that gradually decreases (the step heights are apodized) as the distance of the zone from an optical axis 30 of the lens increases.

By way of example, the step height at each zone boundary of the diffractive pattern can be defined in accordance with the following relation:

$$\text{Step height} = \frac{\lambda}{a(n_2 - n_1)}$$

wherein

$\lambda$ denotes a design wavelength (e.g., 550 nm),
a denotes a parameter that can be adjusted to control diffraction efficiency associated with various orders, e.g., a can be selected to be 2.5,
$n_2$ denotes the index of refraction of the optic,
$n_1$ denotes the refractive index of a medium in which the lens is placed, and $f_{apodize}$ represents a scaling function whose value decreases as a function of increasing distance from the intersection of an optical axis with the anterior surface of the lens. By way of example, the scaling function $f_{apodize}$ can be defined by the following relation:

$$f_{apodize} = 1 - (\frac{r_i}{r_{out}})^3$$

wherein

$r_i$ denotes the radial distance of the $i^{th}$ zone,
$r_{out}$ denotes the outer radius of the last diffractive zone.

Other apodization scaling functions can also be employed, such as those disclosed in a co-pending patent application entitled "Apodized Aspheric Diffractive Lenses," filed December 1, 2004 and having a serial number 11/000770, which is herein incorporated by reference.

The diffractive pattern 26 covers a portion of the anterior surface (it is truncated) and is surrounded by a refractive portion of the surface lacking diffractive structures.

In this embodiment, the lens 22 provides a positive optical power (e.g., an optical power in a range of about 20 D to about 80 D) and the lens 24 provides a negative optical power (e.g., a power in a range of about -5 D to about -60 D), although the signs of the optical powers of the lenses can also be reversed. In many embodiments, the materials from which the lenses 22 and 24 are formed are selected to have sufficiently different refractive dispersions so as to allow compensating for the natural chromatic aberrations of the eye at the far focus. By way of example, the materials described above in connection with the previous embodiments can be utilized to form the lenses 22 and 24. For example, in one embodiment, the lens 22 can be formed of Acrysof lens material and the lens 24 can be formed of Sensar lens material.

In some embodiments, the ophthalmic lens 20 is configured to provide partial compensation of the eye's chromatic aberration. For example, it can be adapted, in a manner discussed above, to correct about 50% of the eye's longitudinal chromatic aberration. In other embodiments, the lens 20 is adapted to provide a full compensation for the eye's chromatic aberrations.

In some embodiments, multi-optic intraocular systems are disclosed that not only provide compensation for natural chromatic aberrations of the eye but also provide some degree of accommodation, e.g., in a range of about 1 to about 6 Diopters. By way of example, FIGURES 10A, 10B and 10C schematically depicts an IOL 100 comprising a posterior optic 102 and an anterior optic 104. The posterior optic includes clasps 106 that contain sockets 108 defined by a latch 110. The anterior optic 104 includes a pair of haptics 112 that are connected to the optic 104 by hinge regions 114 and contain locking pins 116 that are sized and shaped to fit within socket 108. Once implanted in the eye, the contraction of capsular bag can cause compression of the optic 104. As the optic 104 is compressed, the hinges 114 allow the optic 104 to move anteriorly away from the optic 102, with locking pins pivoting within sockets 108. This can change the overall power of the lens system, e.g., in a range of about 1 D to about 6 D. Further details regarding various structural features of accommodative lenses, such as lens 100, can be found in U.S. Patent Application No. 6,969,403. In this embodiment, however, the materials forming the optics 102 and 104 and the curvatures of the optics are selected, in a manner discussed above, to compensate for the natural chromatic aberrations of the eye. For example, the optic 104 can have a positive optical power and the optic 102 can have a negative optical power. Further, the optics 102 and 104 can be formed of materials having sufficiently different Abbe numbers so as to provide a desired chromatic compensation. In general, the degree of the chromatic compensation can vary as the distance between the two optics changes to provide accommodation.

Those having ordinary skill in the art will appreciate that various modifications can be made to the above embodiments without departing from the scope of the invention.

## Claims

**1.** An intraocular lens (10), comprising

a posterior optic (12), and
an anterior optic (14),

wherein said posterior and anterior optics have different chromatic dispersions adapted to cooperatively provide compensation for natural chromatic aberrations of the eye over a wavelength range.

**2.** The intraocular lens of claim 1,
wherein the posterior optic (12) exhibits a refractive dispersion over a wavelength range,
and wherein the anterior optic (14) exhibits a different refractive dispersion over said wavelength range,
and wherein said optics have a plurality of curved surfaces,
and wherein curvatures of the surfaces and the refractive dispersions of the optics are adapted to generate chromatic focal shifts for compensating chromatic aberrations of the eye over said wavelength range.

**3.** The intraocular lens of claim 1 or claim 2, wherein said wavelength range extends from a wavelength of 400 nm to 700 nm.

**4.** The intraocular lens of claim 3, wherein said wavelength range is centered about a wavelength of about 550 nm.

**5.** The intraocular lens of any of claims 1 to 4, wherein said posterior and anterior optics are adapted to collectively provide a chromatic aberration correction in a range of 0.5 Diopters to 3.5 Diopters.

**6.** The intraocular lens of any of claims 1 to 5, wherein one of said optics (12,14) provides a positive optical power and the other optic provides a negative optical power.

**7.** The intraocular lens of claim 6, wherein said posterior optic (12) provides a positive optical power and said anterior optic (14) provides a negative optical power.

**8.** The intraocular lens of any of claims 1 to 7, wherein said optics (12,14) are axially separated by a distance in a range of 0 to 5 millimeters.

**9.** The intraocular lens of any of claims 1 to 8, wherein an optical axis (30) of said posterior optic (12) is substantially coincident with an optical axis (30) of said anterior optic (14).

**10.** The intraocular lens any of claims 1 to 9, wherein said posterior and anterior optics (12,14) collectively provide an optical power in a range of -15 Diopters to 50 Diopters.

**11.** The intraocular lens of claim 10, wherein said posterior and anterior optics (12,14) collectively provide an optical power in a range of 6 Diopters to 34 Diopters.

**12.** The intraocular lens of any of claims 1 to 11, wherein said optics (12,14) are formed of the same or different biocompatible materials.

**13.** The intraocular lens of any of claims 1 to 12, wherein one of said posterior and anterior optics (12,14) is formed of cross-linked copolymer of 2-phenyltheyl acrylate and 2-phenylethyl methacrylate and the other is formed of cross-linked terpolymer of ethyl acrylate, ethylmethacrylate, and 2,2,2-trifluoroethyl methacrylate.

**14.** An intraocular lens system (100) according to any of claims 1 to 13, wherein the posterior optic (102) and the anterior optic (104) are adapted to be movably coupled to one another so as to allow movement of at least one optic, in response to application of a compressive force thereto, relative to the other,
wherein said optics (102,104) have different chromatic dispersions adapted to cooperatively provide compensation for natural chromatic aberrations of the eye over said wavelength range.

**15.** An ophthalmic lens system (20), comprising

a posterior lens (24), and
an anterior lens (22),
a diffractive pattern (26) disposed on a surface of one of said anterior or posterior lenses so as to enable said lenses to cooperatively provide a near focus and a far focus,

wherein said lenses exhibit different chromatic dispersions adapted to compensate for chromatic aberration of the eye at said far focus over a wavelength range.

**16.** The ophthalmic lens of claim 15, wherein said diffractive pattern (26) is an apodized diffractive pattern (28).

**17.** The ophthalmic lens of claim 15 or claim 16, wherein said diffractive pattern provides an add power in a range of 1 to 6 D for generating said near focus.

**18.** The ophthalmic lens of any of claims 15 to 17, wherein one of said posterior and anterior lenses (24,22) is formed of a cross-linked copolymer of 2-phenylethyl acrylate and 2-phenylethyl methacrylate and the other is formed of a cross-linked teropolymer of ethyl acrylate, ethylmethacrylate, and 2,2,2-trifluoroethyl methacrylate.

# Fig. 1

# Fig. 2

# Fig. 3

## Fig. 4

| Eye w/ RTH IOL Polychromatic Diffraction MTF | FBY 0 FBX 0 FOC 0 Tan+ Sag× Limit● |
|---|---|

## Fig. 5

| Eye w/ Spherical BCX IOL Modulation Transfer Function - WV1 | FBY 0 FBX 0 FOC 0 Tan+ Sag× Limit● |
|---|---|

## Fig. 6

| Acromat in Eye, PSFX Focal Length=16.5 NA=0.1536 | Units: mm Des:DRC |
|---|---|

## Fig. 7

| Acromat in Eye, PSFX Polychromatic Diffraction MTF | FBY 0 FBX 0 FOC 0 Tan+ Sag× Limit● |
|---|---|

## Fig. 8

| Acromat in Eye Polychromatic Diffraction MTF | FBY 0 FBX 0 FOC 0 Tan+ Sag× Limit● |
|---|---|

## Fig. 9

### 4.5 mm pupil, MTF=0.381 at 100 lp/mm
21.00 achro, ovg eye - Apodization MTF

Order O, Pupil Diam 4.5 mm, Focus SF 0.0
P-V; 0.237; RMS; 0.054 [waves 0.550 $\mu$m]

FBY 0 FBX 0          Limit +    MTF ●
Cutoff Frequency 559.788374 lp/mm

**Fig. 10**

**Fig. 11A**

**Fig. 11B**

**Fig. 11C**

EP 1 862 147 A1

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 329 981 A (ADVANCE MEDICAL S R L [IT]) 30 August 1989 (1989-08-30) | 1-13 | INV. A61F2/16 |
| Y | * column 2, line 12 - column 3, line 10; figures 5,7 * | 14 | |
| X | WO 2005/098518 A (ADVANCED MEDICAL OPTICS INC [US]; PIERS PATRICIA ANN [NL]; WEEBER HENK) 20 October 2005 (2005-10-20) | 1,3-5, 9-11 | |
| Y | * claim 1; figure 1; example 1 * | 15-18 | |
| X | EP 0 470 811 A2 (MINNESOTA MINING & MFG [US]) 12 February 1992 (1992-02-12) | 1,3-5, 9-11 | |
| Y | * page 3, line 6 - line 15; claim 3; figure 5 * | 15-18 | |
| Y | WO 03/000154 A2 (MILLER DAVID [US]; BLANCO ERNESTO [US]; MAGNANTE PETER [US]) 3 January 2003 (2003-01-03) * claims 1,2,10,20; figures 1,5,6 * | 14-18 | |
| Y | US 5 699 142 A (LEE CHUN-SHEN [US] ET AL) 16 December 1997 (1997-12-16) * claim 1 * | 16 | TECHNICAL FIELDS SEARCHED (IPC) A61F G02C |

The present search report has been drawn up for all claims

| Place of search | Date of completion of the search | Examiner |
|---|---|---|
| Munich | 22 August 2007 | Jestl, Markus |

EPO FORM 1503 03.82 (P04C01)

## ANNEX TO THE EUROPEAN SEARCH REPORT
## ON EUROPEAN PATENT APPLICATION NO.

EP 07 10 8530

This annex lists the patent family members relating to the patent documents cited in the above-mentioned European search report.
The members are as contained in the European Patent Office EDP file on
The European Patent Office is in no way liable for these particulars which are merely given for the purpose of information.

22-08-2007

| Patent document cited in search report | | Publication date | Patent family member(s) | | Publication date |
|---|---|---|---|---|---|
| EP 0329981 | A | 30-08-1989 | IT | 1215851 B | 22-02-1990 |
| WO 2005098518 | A | 20-10-2005 | AU | 2005230194 A1 | 20-10-2005 |
| | | | CA | 2562268 A1 | 20-10-2005 |
| EP 0470811 | A2 | 12-02-1992 | AU | 657094 B2 | 02-03-1995 |
| | | | AU | 8131991 A | 13-02-1992 |
| | | | CA | 2048013 A1 | 09-02-1992 |
| | | | DE | 69127734 D1 | 30-10-1997 |
| | | | DE | 69127734 T2 | 19-03-1998 |
| | | | DK | 470811 T3 | 11-05-1998 |
| | | | ES | 2106766 T3 | 16-11-1997 |
| | | | JP | 3294291 B2 | 24-06-2002 |
| | | | JP | 4254817 A | 10-09-1992 |
| | | | US | 5229797 A | 20-07-1993 |
| WO 03000154 | A2 | 03-01-2003 | EP | 1399097 A2 | 24-03-2004 |
| US 5699142 | A | 16-12-1997 | NONE | | |

EPO FORM P0459

For more details about this annex : see Official Journal of the European Patent Office, No. 12/82

**EP 1 862 147 A1**

## REFERENCES CITED IN THE DESCRIPTION

### Patent documents cited in the description

- US 4834750 A
- US 6969403 A